# EUROPEAN PATENT SPECIFICATION

(11) **EP 3 241 492 B1**
(45) Date of publication and mention of the grant of the patent: **22.07.2020**
(21) Application number: 16874261.7
(22) Date of filing: 21.01.2016
(51) Int. Cl.: A61B 5/024

(54) **HEART RATE DETECTION METHOD AND DEVICE**
VERFAHREN UND VORRICHTUNG ZUR HERZFREQUENZDETEKTION
PROCÉDÉ ET DISPOSITIF DE DÉTECTION DE FRÉQUENCE CARDIAQUE

(30) Priority: 18.12.2015 CN 201510957440
(43) Date of publication of application: 08.11.2017
(73) Proprietor: Shenzhen Goodix Technology Co., Ltd., Futian Free Trade Zone Shenzhen, Guangdong 518000 (CN)
(72) Inventor: YU, Yi, Shenzhen Guangdong 518000 (CN); YANG, Wangwang, Shenzhen Guangdong 518000 (CN); LI, Shunzhan, Shenzhen Guangdong 518000 (CN)
(74) Representative: Sticht, Andreas
(86) International application number: PCT/CN2016/071684
(87) International publication number: WO 2017/101195

(56) References cited:
- EP-A1- 0 925 757
- EP-A1- 2 755 551
- WO-A1-2015/036289
- CN-A- 101 272 731
- CN-A- 102 764 111
- CN-A- 103 781 414
- CN-A- 104 683 581
- US-A1- 2014 073 486
- US-A1- 2014 275 852
- US-A1- 2014 296 658

## Description

### TECHNICAL FIELD

The present invention relates to the technical field of electronics, and in particular, relates to a heart rate detection method and a heart rate detection apparatus.

### BACKGROUND

Heart rate, as important information reflecting human health, is highly concerned by people. Medical heart rate detection is based on electrocardiogram (ECG) signals. Such detection is complicated and need collaboration from others, and moreover, a device for performing the detection is not easy to carry.

In recent years, wearable and handheld smart terminals support users to press cameras with fingers, and detect the heart rate by means of causing the light having a specific wavelength (for example, the red light having a wavelength of 660 nm to 720 nm) to irradiate the fingers of the users and acquiring photoplethysmogram (PPG) signals via the cameras. Such smart terminals as bracelets and mobile phones have good carry convenience and are thus facilitate real-time detection. Currently, more and more smart terminals are supporting this function.

However, most smart terminals, which implement heart rate detection based on PPG signals are defective in that the calculation time is long or/and the calculation error is great.

US patent application US2014/296658A1 discloses an activity tracking device. The activity tracking device is configured to identify heart beats of the user and produce an indication of a heart rate. The activity tracking device further includes a pressure detecting system configured for detecting a pressure applied to the heart rate monitor location on the housing with the skin of the user during the identification of heart beats. The activity tracking device also includes a motion sensor to identify and filter our the falsely detected heart beats from the detected heart beats.

European patent 0925757A1 discloses a pulse counter configured to carry out frequency analysis of a pulse wave signal detected by a pulse wave detecting means.

European patent 2755551A1 discloses a portable device configured to calculate the heart rate based on the heart rate signal if the signal quality is above a predefined threshold, and to estimate the heart rate based on the motion signal if the signal quality is below the threshold.

US patent application US2014/0275852A1 is directed to how to determine the transitions between awake and sleep by observing an increase or decrease in the user's heart rate.

### SUMMARY

Main objectives of the present invention are to provide a heart rate detection method and a heart rate detection apparatus to solve the technical problem that efficiency of detecting a heart rate is low and the error rate is high.

To achieve the above objective, the present invention provides a heart rate detection method according to claim 1.

Optionally, the heart rate detection method further includes: generating first prompt information when it is judged that the heart rate characteristic indicates that the finger of the user fails to completely press a light source for detecting the heart rate; wherein the first prompt information is used for prompting the user to press the finger onto the light source.

Optionally, in the heart rate detection method, the judging whether the heart rate characteristic satisfies a predetermined condition specifically includes: judging whether the heart rate characteristic indicates that a user is in a resting state.

Optionally, in the heart rate detection method, heart rate characteristic includes slopes of an upper wave edge and a lower wave edge of at least one wave in the waveform, and/or a difference between alternating current AC components of at least two wave ridges in the waveform, wherein the alternating current AC component of each wave ridge is an average value of amplitudes of two neighboring wave valleys; and the judging whether the heart rate characteristic indicates that a user is in a resting state specifically includes: judging whether an absolute value of the slope of the upper wave edge is greater than an absolute value of the slope of the lower wave edge; and/or judging whether the difference between the alternating current AC components of the at least two wave ridges falls within a second predetermined interval.

Optionally, the heart rate detection method further includes: generating second prompt information when it is judged that the heart rate characteristic indicates that the user is not in the resting state; wherein the second prompt information is used for prompting the user to retain in the resting state.

To achieve the above objectives, the present invention further provides a heart rate detection apparatus according to claim 6.

Optionally, the heart rate detection apparatus further includes: a first prompt information generating module, configured to generate first prompt information when it is judged that the heart rate characteristic indicates that the finger of the user fails to completely press a light source for detecting the heart rate; wherein the first prompt information is used for prompting the user to press the finger onto the light source.

Optionally, in the heart rate detection apparatus, the judging module is further configured to judge whether the heart rate characteristic indicates that a user is in a resting state.

Optionally, in the heart rate detection apparatus, the heart rate characteristic includes slopes of an upper wave edge and a lower wave edge of at least one wave in the waveform, and/or a difference between alternating current AC components of at least two wave ridges in the waveform, wherein the alternating current AC component of each wave ridge is an average value of amplitudes of two neighboring wave valleys; and the judging module is further configured to judge whether an absolute value of the slope of the upper wave edge is greater than an absolute value of the slope of the lower wave edge; and/or judge whether the difference between the alternating current AC components of the at least two wave ridges falls within a second predetermined interval.

Optionally, the heart rate detection apparatus further includes: a second prompt information generating module, configured to generate second prompt information when it is judged that the heart rate characteristic indicates that the user is not in the resting state; wherein the second prompt information is used for prompting the user to retain in the resting state.

It may be known from the above technical solutions that the heart rate detection method and the heart rate detection apparatus at least have the following advantages.

In the technical solutions according to the present invention, since a waveform of a heart rate signal normally detected has some common features, whether detection of the heart rate is in a normal condition may be judged based on the feature of the waveform. In the normal condition, the waveform of the heart rate signal is stable, and therefore it is unnecessary to detect for a long period whether the waveform is changed. In addition, a correct heart rate value is obtained if calculation of the heart rate value is based on the heart rate signal in the normal condition, which prevents an error to occur.

### BRIEF DESCRIPTION OF THE DRAWINGS

FIG. 1 is a flowchart of a heart rate detection method according to an embodiment of the present invention;
FIG. 2A is a schematic diagram of a waveform of a heart rate signal;
FIG. 2B is a schematic diagram of a waveform of a heart rate signal
FIG. 3 is a flowchart of a heart rate detection method according to an embodiment of the present invention;
FIG. 4 is a schematic diagram of a waveform of another heart rate signal;
FIG. 5 is still a schematic diagram of a waveform of still another heart rate signal
FIG. 6 is a flowchart of a heart rate detection method according to an embodiment of the present invention;
FIG. 7 is a block diagram of a heart rate detection apparatus according to an embodiment of the present invention; and
FIG. 8 is a block diagram of a heart rate detection apparatus according to an embodiment of the present invention.

The attainment of the objectives, functional features and advantages of the present invention are further described hereinafter with reference to the specific embodiments and the accompanying drawings.

### DETAILED DESCRIPTION

It should be understood that the embodiments described here are only exemplary ones for illustrating the present invention, and are not intended to limit the present invention.

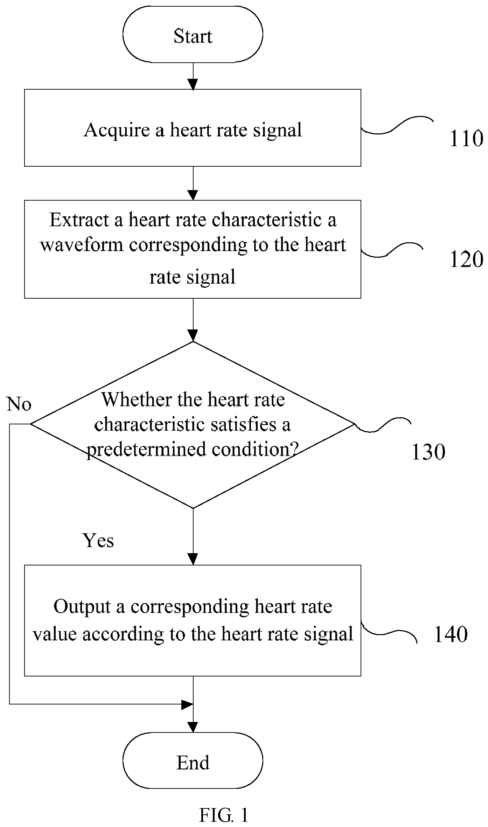

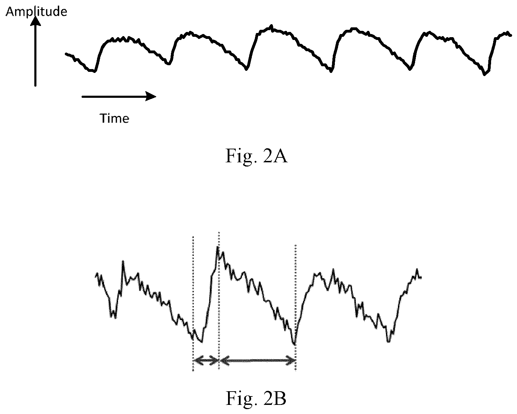

As illustrated in FIG. 1, an embodiment of the present invention provides a heart rate detection method, and the method includes the following steps:
Step 110: A heart rate signal is acquired. In this embodiment, the manner of acquiring the heart rate signal is not limited. For example, a PPG signal is acquired via a camera of a mobile phone to detect the heart rate.
Step 120: A heart rate characteristic is extracted from a waveform corresponding to the heart rate signal. In this embodiment, the type of the extracted heart rate characteristic is not limited, and any characteristic based on waveform is applicable to the technical solution according to embodiment.
Step 130: Whether the heart rate characteristic satisfies a predetermined condition is judged. In this embodiment, a predetermined condition may be any condition complying with a normal heart rate waveform, for example, a stable heart rate signal as illustrated in FIG. 2A, wherein the stable heart rate signal has two characteristics: 1) the wave is asymmetric, that is, absolute values of slopes of an upper wave edge and a lower wave edge (partition of the upper and lower wave edges is as illustrated in FIG. 2B) are different; and 2) the amplitude is stable: in a stable test duration, the amplitude (that is, a wave ridge value) will not be suddenly changed within a specific range. Therefore, conditions set based on these two features are all applicable to the technical solution according to this embodiment. A key point of this embodiment is to elaborately analyze the characteristic of the heart rate signal, and further judge whether the heart rate signal is in a normal condition.
Step 140: A corresponding heart rate value is output according to the heart rate signal when it is judged that the heart rate characteristic satisfies the predetermined condition.

In an existing bracelet, the heart rate can only be obtained and displayed upon a critical condition that the bracelet is kept in a resting state for at least 8 seconds, and with respect to a smart phone for detecting the heart rate by pressing a camera thereof, a calculation result with a larger error may be caused due to a slight hand movement. In the technical solution according to this embodiment, whether detection of the heart rate is in a normal condition may be judged based on the feature of the waveform. In the normal condition, the waveform of the heart rate signal is stable, and therefore it is unnecessary to detect for a long period whether the waveform is changed. In addition, a correct heart rate value is obtained if calculation of the heart rate value is based on the heart rate signal in the normal condition, which prevents an error to occur.

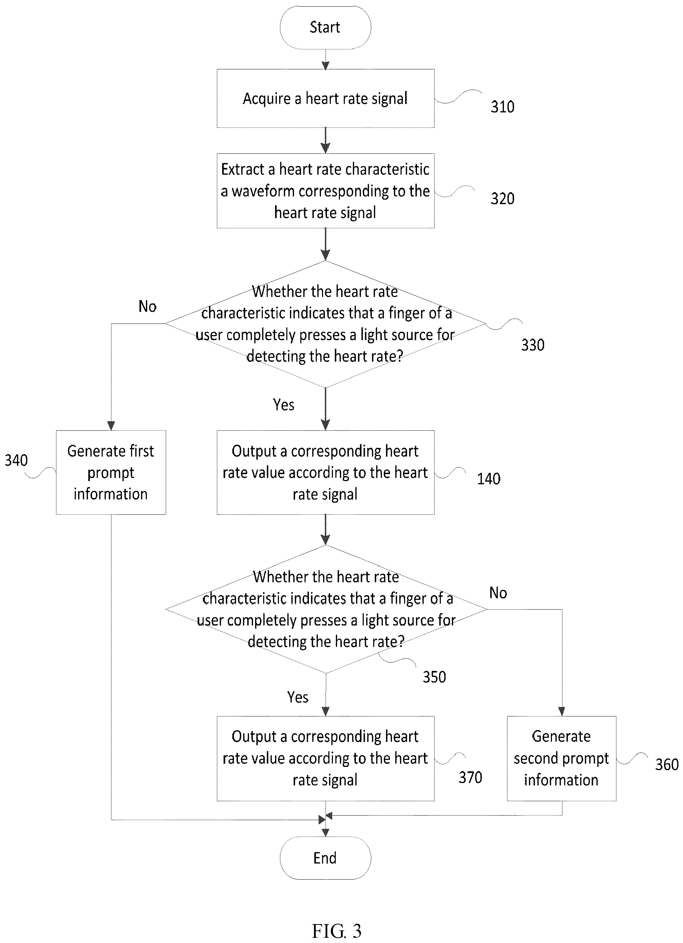

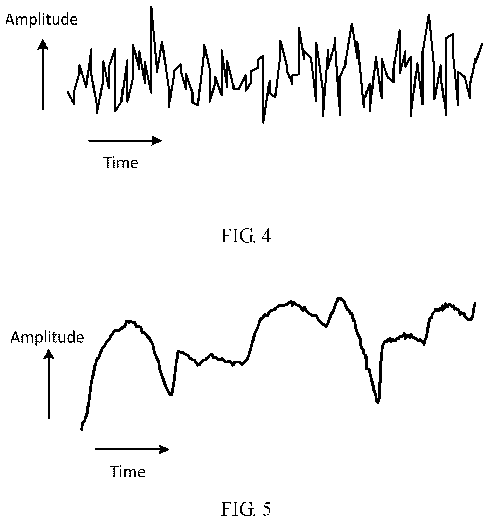

As illustrated in FIG. 3, an embodiment of the present invention provides a heart rate detection method, and the method includes the following steps:
Step 310: A heart rate signal is acquired.
Step 320: A heart rate characteristic is extracted from a waveform corresponding to the heart rate signal.
Step 330: Whether the heart rate characteristic indicates that a finger of a user completely presses a light source for detecting the heart rate is judged. In this embodiment, if the heart rate is detected by using a mobile phone, the light source is a flash light of the mobile phone. When the finger of the user does not completely press the light source, during acquisition of the heart rate signal, interference caused by the ambient light is easy to occur. The acquired heart rate signal is as illustrated in FIG. 4, and the signal has the following characteristics: 1) in this case, an acquired wave ridge value is lower than a wave ridge value when the finger completely presses the light source; and 2) within a fixed time period, the number of searched wave ridges and wave valleys is much greater than that in the case where the finger completely presses the light source. Therefore, in this embodiment, whether the signal is a signal generated when the finger of the user does not completely press a light source may be judged.
Step 340: When the heart rate characteristic indicates that the finger of the user fails to completely press the light source, first prompt information is generated, wherein the first prompt information is used for prompting the user to press the finger onto the light source. In this embodiment, the user needs to be immediately prompted when the heart rate signal is abnormal, which may avoid outputting an incorrect heart rate value to the user.
Step 350: Whether the heart rate characteristic indicates that the user is in a resting state is judged when the heart rate characteristic indicates that the finger of the user completely presses the light source. When a user's hand is in a regular or irregular movement, the acquired heart rate signal is as illustrated in FIG. 5, and the features of the signal are as follows: 1) symmetry of the waveform is good; 2) a change to wave ridge values between waves under irregular movement is great, and the wave ridge values between waves are consistent in the regular movement. Therefore, in this embodiment, whether the signal is generated when the user is in a movement state may be judged.
Step 360: When the heart rate characteristic indicates that the user is in a movement state, second prompt information is generated, wherein the second prompt information is used for prompting the user to retain in the resting state. In this embodiment, the user needs to be immediately prompted when the heart rate signal is abnormal, which may avoid outputting an incorrect heart rate value to the user.
Step 370: When the heart rate characteristic indicates that the user is in the resting state, a corresponding heart rate value is output according to the heart rate signal.

In the technical solution according to this embodiment, in practice, judgment is performed on the quality of a heart rate signal as follows: whether a finger completely presses a light source is judged via acquiring the heart rate signal in real time; if a finger completely presses the light source, the signal may either be a normal heart rate signal or be generated when the user is in a movement state (which brings a large calculation error), a corresponding prompt is given when the user does not press the light source or fails to completely press the light source; and further, whether a hand of the user is in the movement state is judged, a corresponding prompt, for example, "please keep non-movement", is given when the user's hand is in the movement state, and a correct heart rate value may be rapidly calculated for the normal heart rate signal (normally, within 4 to 6 seconds). In the technical solution according to this embodiment, heart rate signals in different conditions may be distinguished, whether a current signal reflects a heart rate of a person may be accurately identified, and a heart rate value or prompt information may be given, such that a feedback result is quickly and accurately given to a user.

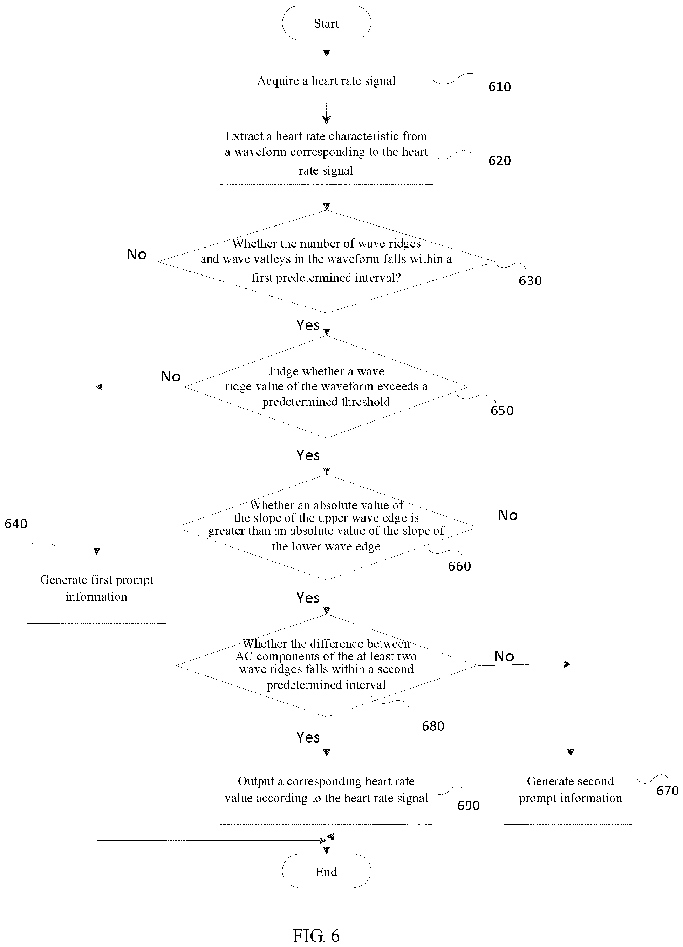

As illustrated in FIG. 6, an embodiment of the present invention provides a heart rate detection method, and the method includes the following steps:
Step 610: A heart rate signal is acquired. In this embodiment, assuming that data acquired per 3 seconds each time is used to perform analysis and judgment (a heart rate of a normal person is 30 BPM to 220 BPM, beat per minute).
Step 620: A heart rate characteristic is extracted from a waveform corresponding to the heart rate signal. The heart rate characteristic includes the number of wave ridges and wave valleys in the waveform, a wave ridge value (i.e., a peak-to-peak value) of the waveform, slopes of an upper wave edge and a lower wave edge of at least one wave in the waveform, and/or a difference between alternating current (AC) components of at least two wave ridges in the waveform, wherein the AC component of each wave ridge is an average value of amplitudes of two neighboring wave valleys.
Step 630: Whether the number of wave ridges and wave valleys in the waveform falls within a first predetermined interval is judged. In this embodiment, the first predetermined interval is not limited. For example, wave valleys are searched for in a waveform of a heart rate signal, and the number of wave ridges and wave valleys appeared in 3 seconds should fall within the range between 1 and 11 (the first predetermined interval). If the number of wave ridges and wave valleys appeared in 3 seconds exceeds 11, it is determined that a light source is not pressed or not completely pressed.
Step 640: When the number of wave ridges and wave valleys in the waveform does not fall within the first predetermined interval, first prompt information is generated, wherein the first prompt information is used for prompting a user to press the finger onto the light source.
Step 650: When the number of wave ridges and wave valleys in the waveform falls within the first predetermined interval, whether a wave ridge value of the waveform exceeds a predetermined threshold is judged. In this embodiment, the predetermined threshold is not limited. For example, according to effects of ambient lights to a heart rate signal, an amplitude difference corresponding to wave ridges and wave valleys is small, that is, if a maximum wave ridge value is smaller than a threshold of 50, it is determined that the light source is not pressed or not completely pressed.

When the wave ridge value of the waveform does not exceed the predetermined threshold, the process returns to step 640 to generate the first prompt information, wherein the first prompt information is used for prompting the user to press the finger onto the light source.
Step 660: Whether an absolute value of the slope of the upper wave edge is greater than an absolute value of the slope of the lower wave edge is judged when the wave ridge value of the waveform exceeds the predetermined threshold. In this embodiment, according to the searched wave valleys, a slope of an upper wave edge and a slope of a lower wave edge of each wave are calculated, and an absolute value of the slope of the upper wave edge of each wave should be greater than an absolute value of the slope of the lower wave edge. If this condition is not satisfied, it is considered that the current signal is a heart rate signal obtained in a relative movement state.
Step 670: Second prompt information is generated when an absolute value of the slope of the upper wave edge is smaller than an absolute value of the slope of the lower wave edge; wherein the second prompt information is used for prompting the user to retain in the resting state.
Step 680: Whether the difference between the AC components of the at least two wave ridges falls within a second predetermined interval is judged when an absolute value of the slope of the upper wave edge is greater than an absolute value of the slope of the lower wave edge. In this embodiment, an AC component of a wave is calculated according to a mean value of amplitudes of a wave valley to a closest wave ridge, and the closest wave ridge to an adjacent wave valley. If a maximum difference value of the AC components is greater than a threshold of 30, it is considered that the current signal is a heart rate signal obtained in a relative movement state.

When the difference does not fall within a second predetermined interval, the process returns to step 670 to generate the second prompt information, wherein the second prompt information is used for prompting the user to retain in the resting state.

Step 690: A corresponding heart rate value is output according to the heart rate signal when the difference falls within a second predetermined interval. If the conditions in the above-described steps are excluded, it is considered that a current signal is a normal heart rate signal, a heart rate value may be calculated and output, wherein the specific calculation method of the heart rate value may be: 60 x sampling rate/mean time of wave valleys.

In the technical solution according to this embodiment, an accurate heart rate result is generally given within 4 to 6 seconds, and compared with an ECG, an error in calculating a heart rate is 5 BPM; moreover, signal quality may be effectively judged to determine whether a heart rate signal is normal, and a corresponding operation prompt is given as necessary.

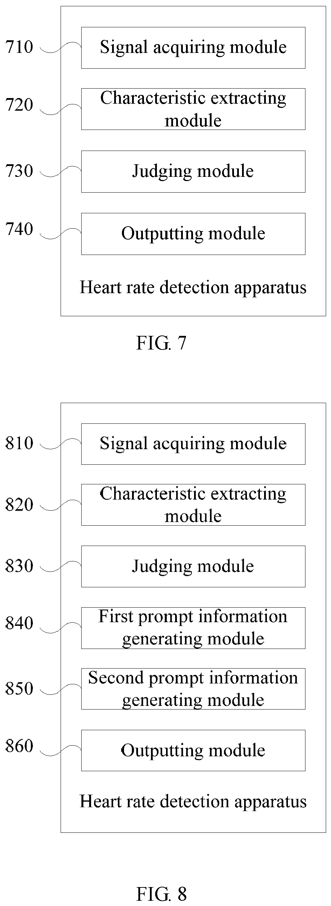

As illustrated in FIG. 7, an embodiment of the present invention provides a heart rate detection apparatus, and the apparatus includes: a signal acquiring module 710, a characteristic extracting module 720, a judging module 730 and an outputting module 740.

The signal acquiring module 710 is configured to acquire a heart rate signal. In this embodiment, the manner of acquiring the heart rate signal is not limited. For example, a PPG signal is acquired via a camera of a mobile phone to detect the heart rate.

The characteristic extracting module 720 is configured to extract a heart rate characteristic from a waveform corresponding to the heart rate signal. In this embodiment, the type of the extracted heart rate characteristic is not limited, and any characteristic based on waveform is applicable to the technical solution according to this embodiment.

The judging module 730 is configured to judge whether the heart rate characteristic satisfies a predetermined condition. In this embodiment, a predetermined condition may be any condition complying with a normal heart rate waveform, for example, a stable heart rate signal as illustrated in FIG. 2A, wherein the stable heart rate signal has two characteristics: 1) the wave is asymmetric, that is, absolute values of slopes of an upper wave edge and a lower wave edge (partition of the upper and lower wave edges is as illustrated in FIG. 2B) are different; and 2) the amplitude is stable: in a stable test duration, the amplitude (that is, a wave ridge value) will not be suddenly changed within a specific range. Therefore, conditions set based on these two features are all applicable to the technical solution according to this embodiment. A key point of this embodiment is to elaborately analyze the characteristic of the heart rate signal, and further judge whether the heart rate signal is in a normal condition.

The outputting module 740 is configured to output a corresponding heart rate value according to the heart rate signal when it is judged that the heart rate characteristic satisfies the predetermined condition.

In an existing bracelet, the heart rate can only be obtained and displayed upon a critical condition that the bracelet is kept in a resting state for at least 8 seconds, and with respect to a smart phone for detecting the heart rate by pressing a camera thereof, a calculation result with a larger error may be caused due to a slight hand movement. In the technical solution according to this embodiment, whether detection of the heart rate is in a normal condition may be judged based on the feature of the waveform. In the normal condition, the waveform of the heart rate signal is stable, and therefore it is unnecessary to detect for a long period whether the waveform is changed. In addition, a correct heart rate value is obtained if calculation of the heart rate value is based on the heart rate signal in the normal condition, which prevents an error to occur.

As illustrated in FIG. 8, an embodiment of the present invention provides a heart rate detection apparatus, and the apparatus includes: a signal acquiring module 810, a characteristic extracting module 820, a judging module 830, a first prompt information generating module 840, a second prompt information generating module 850, and an outputting module 860.

The signal acquiring module 810 is configured to acquire a heart rate signal.

The characteristic extracting module 820 is configured to extract a heart rate characteristic from a waveform corresponding to the heart rate signal.

The judging module 830 is configured to judge whether the heart rate characteristic indicates that a finger of a user completely presses a light source for detecting the heart rate. In this embodiment, if a phone terminal is used to detect the heart rate, the light source is a flash light of a mobile phone. When the finger of the user does not completely press the light source, during acquisition of the heart rate signal, interference caused by the ambient light is easy to occur. The acquired heart rate signal is as illustrated in FIG. 4, and the signal has the following characteristics: 1) in this case, an acquired wave ridge value is lower than a wave ridge value when the finger completely presses the light source; and 2) within a fixed duration, the number of searched wave ridges and wave valleys is much greater than that in the case where the finger completely presses the light source. Therefore, in this embodiment, whether the signal is a signal generated when the finger of the user does not completely press a light source may be judged.

The first prompt information generating module 840 is configured to generate first prompt information when it is judged that the heart rate characteristic indicates that the finger of the user fails to completely press the light source, wherein the first prompt information is used for prompting a user to press the finger onto the light source. In this embodiment, the user needs to be immediately prompted when the heart rate signal is abnormal, which may avoid outputting an incorrect heart rate value to the user.

The judging module 830 is further configured to judge whether the heart rate characteristic indicates that the user is in a resting state when the heart rate characteristic indicates that the finger of the user completely presses a light source. When a user's hand is in a regular or irregular movement, the acquired heart rate signal is as illustrated in FIG. 5, and the signal has the following characteristics: 1) symmetry of the waveform is good; 2) a change to wave ridge values between waves under irregular movement is great, and the wave ridge values between waves are consistent in the regular movement. Therefore, in this embodiment, whether the signal is generated when the user is in a movement state may be judged.

The second prompt information generating module 850 is configured to generate second prompt information when the heart rate characteristic indicates that the user is in a movement state; wherein the second prompt information is used for prompting the user to retain in the resting state. In this embodiment, the user needs to be immediately prompted when the heart rate signal is abnormal, which may avoid outputting an incorrect heart rate value to the user.

The outputting module 860 is configured to output a corresponding heart rate value according to the heart rate signal when the heart rate characteristic indicates that the user is in the resting state.

In the technical solution according to this embodiment, in practice, judgment is performed on the quality of a heart rate signal is judged as follows: whether a finger completely presses a light source is judged via acquiring the heart rate signal in real time; if a finger completely presses the light source, the signal may either be a normal heart rate signal or be generated when the user is in a movement state (which brings a large calculation error), a corresponding prompt is given when the user does not press the light source or fails to completely press the light source; and further, whether a hand of the user is in the movement state is judged, a corresponding prompt for example, "please keep non-movement", is given when the user's hand is in the movement state, and a correct heart rate value may be rapidly calculated for the normal heart rate signal (normally, within 4 to 6 seconds). In the technical solution according to this embodiment, heart rate signals in different conditions may be distinguished, whether a current signal reflects a heart rate of a person may be accurately identified, and a heart rate value or prompt information may be given, such that a feedback result is quickly and accurately given to a user.

An embodiment of the present invention provides a heart rate detection apparatus, and the apparatus includes: a signal acquiring module 810, a characteristic extracting module 820, a judging module 830, a first prompt information generating module 840, a second prompt information generating module 850, and an outputting module 860.

The signal acquiring module 810 is configured to acquire a heart rate signal. In this embodiment, assuming that data acquired per 3 seconds each time is used to perform analysis and judgment (a heart rate of a normal person is 30 BPM to 220 BPM, beat per minute).

The characteristic extracting module 820 is configured to extract a heart rate characteristic from a waveform corresponding to the heart rate signal. The heart rate characteristic includes the number of wave ridges and wave valleys in the waveform, a wave ridge value of the waveform, slopes of an upper wave edge and a lower wave edge of at least one wave in the waveform, and/or a difference between alternating current (AC) components of at least two wave ridges in the waveform, wherein the alternating current AC component of each wave ridge is an average value of amplitudes of two neighboring wave valleys.

The judging module 830 is configured to judge whether the number of wave ridges and wave valleys in the waveform falls within a first predetermined interval. In this embodiment, a first predetermined interval is not limited. For example, wave valleys are searched in a waveform of a heart rate signal, and the number of wave ridges and wave valleys appeared in 3 seconds should fall within the range between 1 and 11 (the first predetermined interval). If the number of wave ridges and wave valleys appeared in 3 seconds exceeds 11, it is determined that a light source is not pressed or not completely pressed.

The first prompt information generating module 840 is configured to generate first prompt information when the number of wave ridges and wave valleys in the waveform does not fall within the first predetermined interval, wherein the first prompt information is used for prompting a user to press the finger onto the light source.

The judging module 830 is further configured to judge whether a wave ridge value of the waveform exceeds a predetermined threshold when the number of wave ridges and wave valleys in the waveform falls within the first predetermined interval. In this embodiment, a predetermined threshold is not limited. For example, according to effects of ambient lights to a heart rate signal, an amplitude difference corresponding to wave ridges and wave valleys is small, that is, if a maximum wave ridge value is smaller than a threshold of 50, it is determined that a light source is not pressed or not completely pressed.

The first prompt information generating module 840 is configured to generate first prompt information when the wave ridge value of the waveform does not exceed the predetermined threshold, wherein the first prompt information is used for prompting a user to press the finger onto the light source.

The judging module 830 is further configured to judge whether an absolute value of the slope of the upper wave edge is greater than an absolute value of the slope of the lower wave edge when the wave ridge value of the waveform exceeds the predetermined threshold. In this embodiment, according to the searched wave valleys, a slope of an upper wave edge and a slope of a lower wave edge of each wave are calculated, and an absolute value of the slope of the upper wave edge of each wave should be greater than an absolute value of the slope of the lower wave edge. If this condition is not satisfied, it is considered that the current signal is a heart rate signal obtained in a relative movement state.

The second prompt information generating module 850 is configured to generate second prompt information when an absolute value of the slope of the upper wave edge is smaller than an absolute value of the slope of the lower wave edge; wherein the second prompt information is used for prompting the user to retain in the resting state.

The judging module 830 is further configured to judge whether the difference between the alternating current AC components of the at least two wave ridges falls within a second predetermined interval when an absolute value of the slope of the upper wave edge is greater than an absolute value of the slope of the lower wave edge. In this embodiment, an AC component of a wave is calculated according to a mean value of amplitudes of a wave valley to a closest wave ridge, and the closest wave ridge to an adjacent wave valley. If a maximum difference value of the AC components is greater than a threshold of 30, it is considered that the current signal is a heart rate signal obtained in a relative movement state.

The second prompt information generating module 850 is further configured to generate second prompt information when the difference does not fall within a second predetermined interval; wherein the second prompt information is used for prompting the user to retain in the resting state.

The outputting module 860 is configured to output a corresponding heart rate value according to the heart rate signal when the difference falls within a second predetermined interval. If the conditions in the above-described steps are excluded, it is considered that the current signal is a normal heart rate signal, a heart rate value may be calculated and output, wherein the specific calculation method of the heart rate value may be: 60 x sampling rate/mean time of wave valleys.

In the technical solution according to this embodiment, an accurate heart rate result is generally given within 4 to 6 seconds, and compared with an ECG, an error in calculating a heart rate is 5 BPM; moreover, signal quality may be effectively judged to determine whether a heart rate signal is normal, and a corresponding operation prompt may be given as necessary.

It should be noted that, in this specification, terms "comprises", "comprising", "has", "having", "includes", "including", "contains", "containing" or any other variation thereof, are intended to cover a non-exclusive inclusion, such that a process, method, article, or apparatus, that comprises, has, includes, contains a list of elements does not include only those elements but may include other elements not expressly listed or inherent to such process, method, article, or apparatus. An element proceeded by "comprises...a", "has...a", "includes...a", "contains...a" does not, without more constraints, preclude the existence of additional identical elements in the process, method, article, or apparatus.

The sequence numbers of the embodiments of the present invention are only for ease of description, but do not denote the preference of the embodiments.

Through the above description of the method embodiments, it is clear to persons skilled in the art that the methods according to the above embodiment may be accomplished by software plus necessary universal hardware platforms, and definitely may also be accomplished by hardware, but in many cases, the software implementation is preferred. Based on such understanding, portions of the technical solutions of the present invention that essentially contribute to the prior art may be embodied in the form of a software product, the computer software product may be stored in a storage medium, such as a ROM/RAM, a magnetic disk, a CD-ROM and the like, including several instructions for causing a computer device (which may be a mobile phone, a personal computer, a server, an air conditioner, a network device or the like) to perform the methods according to various embodiments of the present disclosure.

Described above are preferred embodiments of the present invention, but are not intended to limit the scope of the present invention, which is defined by the claims. Any equivalent structure or equivalent process variation made based on the specification and drawings of the present invention, which is directly or indirectly applied in other related technical fields, fall within the scope of the present disclosure.

## Claims

1. A heart rate detection method carried out in a heart rate detection apparatus, comprising:
acquiring (110, 310, 610) a heart rate signal of a user;
extracting (120, 320, 620) a heart rate characteristic of time domain from a waveform corresponding to the heart rate signal;
judging (330) whether the heart rate characteristic of time domain indicates that a finger of the user completely presses a light source for detecting the heart rate; and
outputting (140, 370, 690) a corresponding heart rate value according to the heart rate signal when it is judged that the heart rate characteristic of time domain indicates that the finger of the user completely presses a light source for detecting the heart rate,
**characterized in that** the heart rate characteristic comprises the number of wave ridges and/or wave valleys in the waveform, and/or a wave ridge value of the waveform, which is defined as a peak-to-peak value of the waveform; and the judging (330) whether the heart rate characteristic indicates whether a finger of a user completely presses a light source for detecting the heart rate comprises:
judging (630) whether the number of wave ridges and/or wave valleys in the waveform falls within a first predetermined numerical range; and/or
judging (650) whether the wave ridge value of the waveform exceeds a predetermined threshold.

2. The heart rate detection method according to claim 1, further comprising:
generating (640) first prompt information when it is judged that the heart rate characteristic indicates that the finger of the user fails to completely press a light source for detecting the heart rate; wherein the first prompt information is used for prompting the user to press the finger onto the light source.

3. The heart rate detection method according to claim 1, the method further comprising: judging (350) whether the heart rate characteristic of time domain indicates that the user is in a resting state when the heart rate characteristic of time domain indicates that the user presses a light source for detecting the heart rate,
when the heart rate characteristic of time domain indicates that the user is in a resting state, perform the operation of outputting (140, 370, 690) a corresponding heart rate value according to the heart rate signal.

4. The heart rate detection method according to claim 3, wherein the heart rate characteristic comprises slopes of an upper wave edge and a lower wave edge of at least one wave in the waveform, and/or a difference between alternating current AC components of at least two wave ridges in the waveform, wherein the alternating current AC component of each wave ridge is an average value of amplitudes of two neighboring wave valleys;
wherein the judging (350) whether the heart rate characteristic indicates that a user is in a resting state comprises:
judging (660) whether an absolute value of the slope of the upper wave edge is greater than an absolute value of the slope of the lower wave edge; and/or
judging (680) whether the difference between the alternating current AC components of the at least two wave ridges falls within a second predetermined numerical range.

5. The heart rate detection method according to claim 3, further comprising:
generating (670) second prompt information when it is judged that the heart rate characteristic indicates that the user is not in the resting state; wherein the second prompt information is used for prompting the user to retain in the resting state.

6. A heart rate detection apparatus, comprising:
a signal acquiring module (710, 810), configured to acquire a heart rate signal of a user;
a characteristic extracting module (720, 820), configured to extract a heart rate characteristic of time domain from a waveform corresponding to the heart rate signal;
a judging module (730, 830), configured to judge whether the heart rate characteristic of time domain indicates that a finger of the user completely presses a light source for detecting the heart rate; and
an outputting module (740, 860), configured to output a corresponding heart rate value according to the heart rate signal when it is judged that the heart rate characteristic of time domain indicates that the finger of the user completely presses a light source for detecting the heart rate.
**characterized in that** the heart rate characteristic comprises the number of wave ridges and/or wave valleys in the waveform, and/or a wave ridge value of the waveform, which is defined as a peak-to-peak value of the waveform; and
the judging module (730, 830) is further configured to judge whether the number of wave ridges and/or wave valleys in the waveform falls within a first predetermined numerical range; and/or judge whether the wave ridge value of the waveform exceeds a predetermined threshold.

7. The heart rate detection apparatus according to claim 6, further comprising:
a first prompt information generating module (840), configured to generate first prompt information when it is judged that the heart rate characteristic indicates that the finger of the user fails to completely press a light source for detecting the heart rate; wherein the first prompt information is used for prompting the user to press the finger onto the light source.

8. The heart rate detection apparatus according to claim 7, the judging module (730, 830) is further configured to:
when the heart rate characteristic of time domain indicates that a finger of the user completely presses a light source by pressing, judge whether the heart rate characteristic of time domain indicates that the user is in a resting state; and
output (140, 370, 690) a corresponding heart rate value according to the heart rate signal when it is judged that the heart rate characteristic of time domain indicates that the user is in a resting state.

9. The heart rate detection apparatus according to claim 8, wherein the heart rate characteristic comprises slopes of an upper wave edge and a lower wave edge of at least one wave in the waveform, and/or a difference between alternating current AC components of at least two wave ridges in the waveform, wherein the alternating current AC component of each wave ridge is an average value of amplitudes of two neighboring wave valleys;
the judging module (730, 830) is further configured to judge whether an absolute value of the slope of the upper wave edge is greater than an absolute value of the slope of the lower wave edge; and/or judge whether the difference between the alternating current AC components of the at least two wave ridges falls within a second predetermined numerical range.

10. The heart rate detection apparatus according to claim 8, further comprising:
a second prompt information generating module (850), configured to generate second prompt information when it is judged that the heart rate characteristic indicates that the user is not in the resting state; wherein the second prompt information is used for prompting the user to retain in the resting state.

## Patentansprüche

1. Herzfrequenz-Erfassungsverfahren, das in einem Herzfrequenz-Erfassungsapparat ausgeführt wird, umfassend:
Erfassen (110, 310, 610) eines Herzfrequenzsignals eines Benutzers;
Extrahieren (120, 320, 620) einer Herzfrequenzcharakteristik in einem Zeitbereich aus einer dem Herzfrequenzsignal entsprechenden Wellenform;
Bestimmen (330), ob die Herzfrequenzcharakteristik im Zeitbereich anzeigt, dass ein Finger des Benutzers eine Lichtquelle zur Erfassung der Herzfrequenz vollständig drückt; und
Ausgeben (140, 370, 690) eines entsprechenden Herzfrequenzwerts gemäß dem Herzfrequenzsignal, wenn es bestimmt wird, dass die Herzfrequenzcharakteristik im Zeitbereich anzeigt, dass der Finger des Benutzers die Lichtquelle zur Erfassung der Herzfrequenz vollständig drückt,
**dadurch gekennzeichnet, dass** die Herzfrequenzcharakteristik die Anzahl von Wellenkämmen und/oder Wellentälern in der Wellenform und/oder einen Wellenkammwert der Wellenform umfasst, der als ein Spitze-Spitze-Wert der Wellenform definiert ist; und das Bestimmen (330), ob die Herzfrequenzcharakteristik anzeigt, dass der Finger des Benutzers die Lichtquelle zur Erfassung der Herzfrequenz vollständig drückt, umfasst:
Bestimmen (630), ob die Anzahl von den Wellenkämmen und/oder den Wellentälern in der Wellenform in einen ersten vorbestimmten Zahlenbereich fällt; und/oder
Bestimmen (650), ob der Wellenkammwert der Wellenform einen vorbestimmten Schwellenwert überschreitet.

2. Herzfrequenz-Erfassungsverfahren nach Anspruch 1, ferner umfassend:
Erzeugen (640) einer ersten Prompt-Information, wenn es bestimmt wird, dass die Herzfrequenzcharakteristik anzeigt, dass der Finger des Benutzers die Lichtquelle zur Erfassung der Herzfrequenz nicht vollständig drückt; wobei die erste Prompt-Information dazu verwendet wird, den Benutzer aufzufordern, den Finger auf die Lichtquelle zu drücken.

3. Herzfrequenz-Erfassungsverfahren nach Anspruch 1, wobei das Verfahren ferner umfasst: Bestimmen (350), ob die Herzfrequenzcharakteristik im Zeitbereichs anzeigt, dass sich der Benutzer in einem Ruhezustand befindet, wenn die Herzfrequenzcharakteristik im Zeitbereich anzeigt, dass der Benutzer die Lichtquelle zur Erfassung der Herzfrequenz drückt,
wenn die Herzfrequenzcharakteristik im Zeitbereich anzeigt, dass sich der Benutzer in einem Ruhezustand befindet, führen die Operation des Ausgebens (140, 370, 690) eines entsprechenden Herzfrequenzwerts gemäß dem Herzfrequenzsignal durch.

4. Herzfrequenz-Erfassungsverfahren nach Anspruch 3, wobei die Herzfrequenzcharakteristik Steigungen einer oberen Wellenkante und einer unteren Wellenkante mindestens einer Welle in der Wellenform und/oder eine Differenz zwischen Wechselstromkomponenten von mindestens zwei Wellenkämmen in der Wellenform umfasst, wobei die Wechselstromkomponente jedes Wellenkamms ein Durchschnittswert der Amplituden zweier benachbarter Wellentäler ist;
wobei das Bestimmen (350), ob die Herzfrequenzcharakteristik anzeigt, dass sich ein Benutzer in einem Ruhezustand befindet, umfasst:
Bestimmen (660), ob ein absoluter Wert der Steigung der oberen Wellenkante größer ist als ein absoluter Wert der Steigung der unteren Wellenkante; und/oder
Bestimmen (680), ob die Differenz zwischen den Wechselstrom-Komponenten von den mindestens zwei Wellenkämmen in einen zweiten vorbestimmten Zahlenbereich fällt.

5. Herzfrequenz-Erfassungsverfahren nach Anspruch 3, ferner umfassend:
Erzeugen (670) einer zweiten Prompt-Information, wenn es bestimmt wird, dass die Herzfrequenzcharakteristik anzeigt, dass der Benutzer nicht im Ruhezustand ist; wobei die zweite Prompt-Information dazu verwendet wird, den Benutzer aufzufordern, im Ruhezustand zu bleiben.

6. Herzfrequenz-Erfassungsapparat, umfassend:
ein Signalerfassungsmodul (710, 810), das konfiguriert ist, um ein Herzfrequenzsignal eines Benutzers zu erfassen;
ein Charakteristik-Extraktionsmodul (720, 820), das konfiguriert ist, um eine Herzfrequenzcharakteristik in einem Zeitbereich aus einer dem Herzfrequenzsignal entsprechenden Wellenform zu extrahieren;
ein Bestimmungsmodul (730, 830), das konfiguriert ist, um zu bestimmen, ob die Herzfrequenzcharakteristik im Zeitbereichs anzeigt, dass ein Finger des Benutzers eine Lichtquelle zur Erfassung der Herzfrequenz vollständig drückt; und
ein Ausgabemodul (740, 860), das konfiguriert ist, um einen entsprechenden Herzfrequenzwert gemäß dem Herzfrequenzsignal auszugeben, wenn es bestimmt wird, dass die Herzfrequenzcharakteristik im Zeitbereich anzeigt, dass der Finger des Benutzers die Lichtquelle zur Erfassung der Herzfrequenz vollständig drückt,
**dadurch gekennzeichnet, dass**
die Herzfrequenzcharakteristik die Anzahl von Wellenkämmen und/oder Wellentälern in der Wellenform und/oder einen Wellenkammwert der Wellenform umfasst, der als ein Spitze-Spitze-Wert der Wellenform definiert ist; und
das Bestimmungsmodul (730, 830) ferner konfiguriert ist, um zu bestimmen, ob die Anzahl von den Wellenkämmen und/oder den Wellentälern in der Wellenform in einen ersten vorbestimmten Zahlenbereich fällt; und/oder um zu bestimmen, ob der Wellenkammwert der Wellenform einen vorbestimmten Schwellenwert überschreitet.

7. Herzfrequenz-Erfassungsapparat nach Anspruch 6, ferner umfassend:
ein erstes Prompt-Informationserzeugungsmodul (840), das konfiguriert ist, um eine erste Prompt-Information zu erzeugen, wenn es bestimmt wird, dass die Herzfrequenzcharakteristik anzeigt, dass der Finger des Benutzers nicht die Lichtquelle zur Erfassung der Herzfrequenz vollständig drückt; wobei die erste Prompt-Information dazu verwendet ist, den Benutzer aufzufordern, den Finger auf die Lichtquelle zu drücken.

8. Herzfrequenz-Erfassungsapparat nach Anspruch 7, wobei das Bestimmungsmodul (730, 830) ferner konfiguriert ist:
um zu bestimmen, ob die Herzfrequenzcharakteristik im Zeitbereich anzeigt, dass sich der Benutzer in einem Ruhezustand befindet, wenn die Herzfrequenzcharakteristik im Zeitbereichs anzeigt, dass der Finger des Benutzers die Lichtquelle durch drücken vollständig drückt; und
um einen entsprechenden Herzfrequenzwert gemäß dem Herzfrequenzsignal auszugeben (140, 370, 690), wenn es bestimmt wird, dass die Herzfrequenzcharakteristik im Zeitbereich anzeigt, dass sich der Benutzer in einem Ruhezustand befindet.

9. Herzfrequenz-Erfassungsapparat nach Anspruch 8, wobei die Herzfrequenzcharakteristik Steigungen einer oberen Wellenkante und einer unteren Wellenkante mindestens einer Welle in der Wellenform und/oder eine Differenz zwischen Wechselstromkomponenten von mindestens zwei Wellenkämmen in der Wellenform umfasst, wobei die Wechselstromkomponente jedes Wellenkamms ein Durchschnittswert der Amplituden zweier benachbarter Wellentäler ist;
das Bestimmungsmodul (730, 830) ferner konfiguriert ist, um zu bestimmen, ob ein absoluter Wert der Steigung der oberen Wellenkante größer ist als ein absoluter Wert der Steigung der unteren Wellenkante; und/oder um zu bestimmen, ob die Differenz zwischen den Wechselstromkomponenten von den mindestens zwei Wellenkämmen in einen zweiten vorbestimmten Zahlenbereich fällt.

10. Herzfrequenz-Erfassungsapparat nach Anspruch 8, ferner umfassend:
ein zweites Prompt-Informationserzeugungsmodul (850), das konfiguriert ist, um eine zweite Prompt-Information zu erzeugen, wenn es bestimmt wird, dass die Herzfrequenzcharakteristik anzeigt, dass sich der Benutzer nicht im Ruhezustand befindet; wobei die zweite Prompt-Information dazu verwendet ist, den Benutzer aufzufordern, im Ruhezustand zu bleiben.

## Revendications

1. Procédé de détection de fréquence cardiaque mis en œuvre dans un appareil de détection de fréquence cardiaque, comprenant:
acquérir (110, 310, 610) un signal de fréquence cardiaque d'un utilisateur;
extraire (120, 320, 620) une caractéristique du domaine temporel de la fréquence cardiaque d'une forme d'onde correspondant au signal de fréquence cardiaque;
apprécier (330) si la caractéristique du domaine temporel de la fréquence cardiaque indique qu'un doigt de l'utilisateur appuie, complètement, sur une source de lumière pour détecter la fréquence cardiaque; et
délivrer (140, 370, 690) une valeur de fréquence cardiaque correspondante en fonction du signal de fréquence cardiaque lorsqu'il est apprécié que la caractéristique du domaine temporel de la fréquence cardiaque indique que le doigt de l'utilisateur appuie, complètement, sur une source lumineuse pour détecter la fréquence cardiaque,
**caractérisé en ce que** la caractéristique de la fréquence cardiaque comprend le nombre de crêtes d'onde et/ou de vallées d'onde dans la forme d'onde, et/ou une valeur de crête d'onde de la forme d'onde, qui est définie comme une valeur crête à crête de la forme d'onde;
et appréciant (330) si la caractéristique de la fréquence cardiaque indique si un doigt de l'utilisateur appuie, complètement, sur une source de lumière pour détecter la fréquence cardiaque comprend:
apprécier(630) si le nombre de crêtes d'onde et/ou de vallées d'onde dans la forme d'onde tombe dans une première plage numérique prédéterminée; et/ou
apprécier(650) si la valeur de crête d'onde de la forme d'onde dépasse un seuil prédéterminé.

2. Procédé de détection de fréquence cardiaque selon la revendication 1, comprenant en outre:
générer (640) des premières informations d'invite lorsqu'il est apprécié que la caractéristique de la fréquence cardiaque indique que le doigt de l'utilisateur ne parvient pas à appuyer, complètement, sur une source de lumière pour détecter la fréquence cardiaque; dans lequel les premières informations d'invite sont utilisées pour inviter l'utilisateur à appuyer le doigt sur la source de lumière.

3. Procédé de détection de fréquence cardiaque selon la revendication 1, le procédé comprenant en outre: apprécier (350) si la caractéristique du domaine temporel de la fréquence cardiaque indique que l'utilisateur est dans un état de repos lorsque la caractéristique du domaine temporel de la fréquence cardiaque indique que l'utilisateur appuie sur une source lumineuse pour détecter la fréquence cardiaque,
lorsque la caractéristique du domaine temporel de la fréquence cardiaque indique que l'utilisateur est dans l'état de repos, effectuer une opération de délivrance (140, 370, 690) d'une valeur de fréquence cardiaque correspondante en fonction du signal de fréquence cardiaque.

4. Procédé de détection de fréquence cardiaque selon la revendication 3, dans lequel la caractéristique de la fréquence cardiaque comprend des pentes d'un bord d'onde supérieur et d'un bord d'onde inférieur d'au moins une onde dans la forme d'onde, et/ou une différence entre des composantes de courant alternatif d'au moins deux crêtes d'onde dans la forme d'onde, dans laquelle la composante de courant alternatif de chaque crête d'onde est une valeur moyenne des amplitudes de deux vallées d'onde voisines;
dans lequel appréciant (350) si la caractéristique de la fréquence cardiaque indique qu'un utilisateur est dans l'état de repos comprend:
apprécier(660) si une valeur absolue de la pente du bord d'onde supérieur est supérieure à une valeur absolue de la pente du bord d'onde inférieur; et/ou
apprécier(680) si la différence entre les composantes de courant alternatif des au moins deux crêtes d'onde se situe dans une seconde plage numérique prédéterminée.

5. Procédé de détection de la fréquence cardiaque selon la revendication 3, comprenant en outre:
générer (670) des secondes informations d'invite lorsqu'il est apprécié que la caractéristique de la fréquence cardiaque indique que l'utilisateur n'est pas dans l'état de repos; dans lequel les secondes informations d'invite sont utilisées pour inviter l'utilisateur à se maintenir dans l'état de repos.

6. Appareil de détection de fréquence cardiaque, comprenant:
un module d'acquisition de signal (710, 810), configuré pour acquérir un signal de fréquence cardiaque d'un utilisateur;
un module d'extraction de caractéristiques (720, 820), configuré pour extraire une caractéristique du domaine temporel de la fréquence cardiaque d'une forme d'onde correspondant au signal de fréquence cardiaque;
un module d'appréciation (730, 830), configuré pour apprécier si la caractéristique du domaine temporel de fréquence cardiaque indique qu'un doigt de l'utilisateur appuie, complètement, sur une source de lumière pour détecter la fréquence cardiaque; et
un module de délivrance (740, 860), configuré pour délivrer une valeur de fréquence cardiaque correspondante en fonction du signal de fréquence cardiaque lorsqu'il est apprécié que la caractéristique du domaine temporel de fréquence cardiaque indique que le doigt de l'utilisateur appuie, complètement, sur une source de lumière pour détecter la fréquence cardiaque.
**caractérisé en ce que** la caractéristique de fréquence cardiaque comprend le nombre de crêtes d'onde et/ou de vallées d'onde dans la forme d'onde, et/ou une valeur de crête d'onde de la forme d'onde, qui est définie comme une valeur crête à crête de la forme d'onde;
et
le module d'appréciation (730, 830) est en outre configuré pour apprécier si le nombre de crêtes d'onde et/ou de vallées d'onde dans la forme d'onde tombe dans une première plage numérique prédéterminée; et/ou capprécier si la valeur de crête d'onde de la forme d'onde dépasse un seuil prédéterminé.

7. Appareil de détection de fréquence cardiaque selon la revendication 6, comprenant en outre:
un premier module de génération d'informations d'invite (840), configuré pour générer des premières informations d'invite lorsqu'il est apprécié que la caractéristique de la fréquence cardiaque indique que le doigt de l'utilisateur ne parvient pas à appuyer, complètement, sur une source de lumière pour détecter la fréquence cardiaque; dans lequel les premières informations d'invite sont utilisées pour inviter l'utilisateur à appuyer le doigt sur la source de lumière.

8. Appareil de détection de fréquence cardiaque selon la revendication 7, le module d'appréciation(730, 830) est en outre configuré pour:
apprécier si la caractéristique du domaine temporel de la fréquence cardiaque indique que l'utilisateur est dans un état de repos lorsque la caractéristique du domaine temporel de la fréquence cardiaque indique qu'un doigt de l'utilisateur appuie, complètement, sur une source de lumière; et
délivrer (140, 370, 690) une valeur de fréquence cardiaque correspondante en fonction du signal de fréquence cardiaque lorsqu'il est apprécié que la caractéristique du domaine temporel de la fréquence cardiaque indique que l'utilisateur est dans l'état de repos.

9. Appareil de détection de fréquence cardiaque selon la revendication 8, dans lequel la caractéristique de la fréquence cardiaque comprend des pentes d'un bord d'onde supérieur et d'un bord d'onde inférieur d'au moins une onde dans la forme d'onde, et/ou une différence entre des composantes de courant alternatif d'au moins au moins deux crêtes d'onde dans la forme d'onde, dans laquelle la composante de courant alternatif de chaque crête d'onde est une valeur moyenne des amplitudes de deux vallées d'onde voisines;
le module d'appréciation(730, 830) est en outre configuré pour apprécier si une valeur absolue de la pente du bord d'onde supérieur est supérieure à une valeur absolue de la pente du bord d'onde inférieur; et/ou apprécier si la différence entre les composantes de courant alternatif des au moins deux crêtes d'onde se situe dans une seconde plage numérique prédéterminée.

10. Appareil de détection de fréquence cardiaque selon la revendication 8, comprenant en outre:
un deuxième module de génération d'informations d'invite (850), configuré pour générer des secondes informations d'invite lorsqu'il est apprécié que la caractéristique de la fréquence cardiaque indique que l'utilisateur n'est pas dans l'état de repos; dans lequel les secondes informations d'invite sont utilisées pour inviter l'utilisateur à se maintenir dans l'état de repos.
